# EUROPÄISCHE PATENTSCHRIFT

(11) **EP 0 400 587 B1**
(45) Veröffentlichungstag und Bekanntmachung des Hinweises auf die Patenterteilung: **07.09.1994**
(21) Anmeldenummer: 90110208.7
(22) Anmeldetag: 30.05.1990
(51) Int. Cl.: G01L 9/00, G01L 27/00

(54) **Druckmessvorrichtung für in Leitungen strömende Fluide**
Pressure measuring device for fluids flowing in pipes
Dispositif de mesure de pression de fluides coulants dans des conduites

(30) Priorität: 31.05.1989 DE 3918142
(43) Veröffentlichungstag der Anmeldung: 05.12.1990
(73) Patentinhaber: Wiest, Peter P., Dipl.-Ing., D-14052 Berlin (DE)
(72) Erfinder: Wiest, Peter P., Dipl.-Ing., D-14052 Berlin (DE)
(74) Vertreter: Lüke, Dierck-Wilm, Dipl.-Ing.

(56) Entgegenhaltungen:
- EP-A- 0 165 740
- CH-A- 502 593
- DE-A- 3 519 837
- US-A- 4 065 969
- US-A- 4 174 637
- US-A- 4 257 260
- US-A- 4 784 648

## Beschreibung

Die Erfindung bezieht sich auf eine Druckmeß- und Überwachungsvorrichtung für in Leitungen strömende Fluide gemäß den Merkmalen des Oberbegriffes des Anspruches 1.

Eine Druckmeß- und Überwachungsvorrichtung dieser Art für in Leitungen strömende Fluide, insbesondere für Blut, ist aus der DE 27 53 346 vorbekannt. Diese ist mit einem in einer Hauptleitung eingesetzten Schlauchkissen, mit einer Haltevorrichtung für das Schlauchkissen mit zwei Druckmeßelementen versehen. Das eine Druckmeßelement besteht aus einem konventionellen Manometer, das in eine Nebenleitung eingesetzt ist, die an die Hauptleitung im Bereich des Schlauchkissens angeschlossen ist und an dem der aktuelle Druck in der Hauptleitung abgelesen wird. Das andere Druckmeßelement ist ein Mikroschalter, der über ein inneres Fühlerrad an das Schlauchkissen angeschlossen und in einen Signalkreis eingeschaltet ist. Dieser wird geschlossen, falls der Druck in der Hauptleitung einen vorherbestimmten Wert unterschreitet, der mit Hilfe des Fühlerrades geregelt werden kann. Bei dieser vorbekannten Vorrichtung dient das an die Hauptleitung angeschlossene konventionelle Manometer zur Kalibrierung des Drucküberwachers, der aus dem Fühlerrad, dem angeschlossenen Mikroschalter und dem Signalkreis besteht. Nachteilig hierbei ist somit, daß mittels dieser vorbekannten Vorrichtung keine genaue Messung und Kontrolle des Strömungsdruckes in der Hauptleitung erfolgen kann, so daß insbesondere keine Regelung des Druckes einer an die Hauptleitung angeschlossenen Pumpe erfolgen kann.

Eine weitere Druckmeß- und Überwachungsvorrichtung für in Leitungen strömende Fluide ist aus der DE-OS 33 38 758 vorbekannt. Der hierbei verwendete Drucksensor kann ein Stethem-Element sein, das mit einem festen Druckdom mit zwei Anschlüssen für die Schlauchleitung und mit einer den Druckdom abschließenden Membrane versehen ist. Mittels einer Überwurfmutter ist der feste Druckdom mit seiner Membrane gegen einen Flächendruckaufnehmer gedrückt. Der Drucksensor ist an der der Membrane gegenüberliegenden Druckfläche angebracht. Als Drucksensor können Halbleitersensoren oder Dehnungsmeßstreifen dienen.

Bekannt ist auch die Übertragung des Fluiddruckes über ein Sterilfilter vom sterilen Schlauchbesteck auf einen nicht sterilen Drucksensor mittels einer Luftsäule. Auch sterile Einmal-Druckaufnehmer oder resterilisierbare Druckaufnehmer können verwendet werden, welche von dem strömenden Fluid durchflossen werden. Mittels des Drucksensors wird einerseits der in der Körperhöhle aufgebaute Druck erfaßt und andererseits wird danach die Drehzahl der Rollenpumpe geregelt, auf deren Zulaufseite nur ein Begrenzer für einen Maximaldruckwert vorgesehen ist.

Nachteilig ist bei dieser bekannten Druckmeßvorrichtung, daß die Druckregelung der Pumpe dann versagt, wenn der Drucksensor oder deren Auswerteelektronik den ersten Fehler aufweist. Ein solcher Fehler kann z.B. darin liegen, daß der Drucksensor einen zu geringen Systemdruck anzeigt, obwohl der tatsächliche Systemdruck erheblich größer und damit gefährlich für den Patienten ist. Mit der vorbekannten Druckmeßvorrichtung erfolgt somit nur eine Messung des tatsächlichen Druckes, ohne daß eine Kontrolle erfolgt, ob der tatsächlich gemessene Druck auch der richtige Systemdruck ist.

Bei der Druckübertragung durch eine Luftsäule ist zusätzlich durch eine mögliche Undichtigkeit zwischen dem Sterilfilter und dem Drucksensor eine weitere Fehlerquelle vorhanden.

Um die bekannte Druckmeßvorrichtung sicherer auszugestalten, ist zusätzlich eine mechanische Überdrucksicherung in der Leitung angeordnet worden. Dies erfordert jedoch zusätzliche Sterilitätsmaßnahmen und hat den weiteren Nachteil eines möglichen Verklebens des Überdrucksicherungsventiles z.B. bei Verwendung zuckerhaltiger Lösungen. Außerdem wird im Falle eines Überdruckes das in der Leitung strömende Fluid in die Atmosphäre abgelassen, wodurch bei der Behandlung eines Patienten eine erhebliche Unsauberkeit des Behandlungsplatzes auftritt.

Die Verwendung eines zusätzlichen Überdrucksicherheitsventiles, die Verwendung von Einmaldruckaufnehmern und die Verwendung resterilisierbarer Druckaufnehmer hat regelmäßig einen erhöhten Arbeitsaufwand zur Folge, so daß deren Verwendung unwirtschaftlich ist.

Der Erfindung liegt die Aufgabe zugrunde, eine Druckmeß- und Überwachungsvorrichtung der gattungsgemäßen Art zu schaffen, welche gleichzeitig mit der Druckmessung eine unabhängige Überwachung des gemessenen Druckes in einfacher Weise ermöglicht, ohne daß ein großer Bauaufwand benötigt wird.

Die Lösung dieser Aufgabe ergibt sich aus den kennzeichnenden Merkmalen des Anspruches 1. Erfindungsgemäß wird jeder Membrane des Schlauchkissens ein Drucksensor zugeordnet, von denen einer zur Messung des Fluiddruckes und der andere zur Meßwertüberwachung dienen. Ein gegebenenfalls auftretender Differenzdruck zwischen den von beiden unabhängig arbeitenden Drucksensoren ermittelten Drücken zeigt unmittelbar an, daß ein Meßfehler vorliegt, der z.B. zum sofortigen Abstellen einer Pumpe führt, wobei die Verwendung eines Überdrucksicherheitsventiles nicht notwendig ist.

Darüber hinaus sieht die Erfindung eine einfache Anordnung des Schlauchkissens als Bestandteil eines Einmalschlauchstückes in einer Haltevorrichtung vor, in deren beiden Stützwänden jeweils ein Druckmeßsensor als Flächendruckmesser angeordnet ist, mit welchem die Bewegung der jeweiligen Membrane direkt gemessen wird. Die Drucksensoren sind jeweils unmittelbar in die Halterung eingebaut. Da die Schlauchstücke als Einwegprodukte hergestellt werden können und die übrigen Bauelemente der Vorrichtung nicht sterilisiert werden müssen, ist die Druckmeßvorrichtung äußerst einfach in der Handhabung und preiswert in der Herstellung.

Mit der erfindungsgemäßen Vorrichtung werden zeit- und phasengleich sowohl eine Druckmessung des aktuellen Druckes in der Leitung als auch eine Drucküberwachung durchgeführt und in der Anzeigeelektronik ausgewertet, so daß ein eventueller Fehler in der Druckmessung bzw. unzulässig hoher oder unzulässig niedriger Druck sofort angezeigt werden können. Die zeit- und phasengleiche Messung mit beiden Drucksensoren wird dadurch ermöglicht, daß diese auf gegenüberliegenden Membranen des Schlauchkissens jeweils symmetrisch zur Membranfläche angeordnet sind und daher regelmäßig die gleichen Drücke anzeigen.

Gemäß der Erfindung wird ein Schlauchstück mit einem Schlauchkissen aus zwei parallel zueinander und im Abstand voneinander angeordneten Membranen verwendet, wobei jeder Membran ein Drucksensor zugeordnet ist. Hierdurch wird zeit- und phasengleich mit voneinander unabhängigen Drucksensoren, die jeder Membrane zugeordnet sind, einerseits der Druckwert selbst gemessen und andererseits der Druckwert zusätzlich überwacht, indem in der Auswerteelektronik regelmäßig ein eventueller Differenzdruck angezeigt und ausgewertet wird, der zum Stillsetzen der Pumpe führen kann. Die Erfindung besitzt somit die wesentliche Grundbauform eines Schlauchstückes mit einem integrierten Schlauchkissen aus zwei parallelen Membranen. Diese sollen möglichst dünn und hochelastisch sein, wobei eine Dicke von ca. 0,5 mm bevorzugt ist, wobei die übrigen Teile der Schlauchleitung eine Wandstärke von minimal 1,5 mm aufweisen sollten. Nach der Erfindung kann jedoch auch ein starres Innenrohr zwischen den flexiblen Membranen angeordnet sein.

Weitere vorteilhafte Ausgestaltungen der Erfindung ergeben sich aus den weiteren Unteransprüchen.

Die Erfindung ist nachfolgend anhand von zwei in den Zeichnungen dargestellten Ausführungsbeispielen eines Schlauchstückes mit einem Schlauchkissen und anhand einer konkreten Ausführungsform einer Druckmeß- und Überwachungsvorrichtung näher erläutert. Es zeigen:
- Fig. 1: einen Längsschnitt durch ein mit einem Schlauchkissen versehenes Schlauchstück in der ersten Ausführungsform,
- Fig. 2: einen Längsschnitt durch ein in Schlauchstück eingesetztes Schlauchkissen in der zweiten Ausführungsform,
- Fig. 3: eine Draufsicht auf eine Druckmeßvorrichtung,
- Fig. 4: einen Querschnitt durch die Druckmeßvorrichtung gemäß Linie IV-IV in Fig. 3,
- Fig. 5: eine Draufsicht auf eine Rollenpumpe mit daran befindlicher Druckmeßvorrichtung und
- Fig. 6: eine Prinzipdarstellung eines Schaltplanes der Auswerteelektronik.

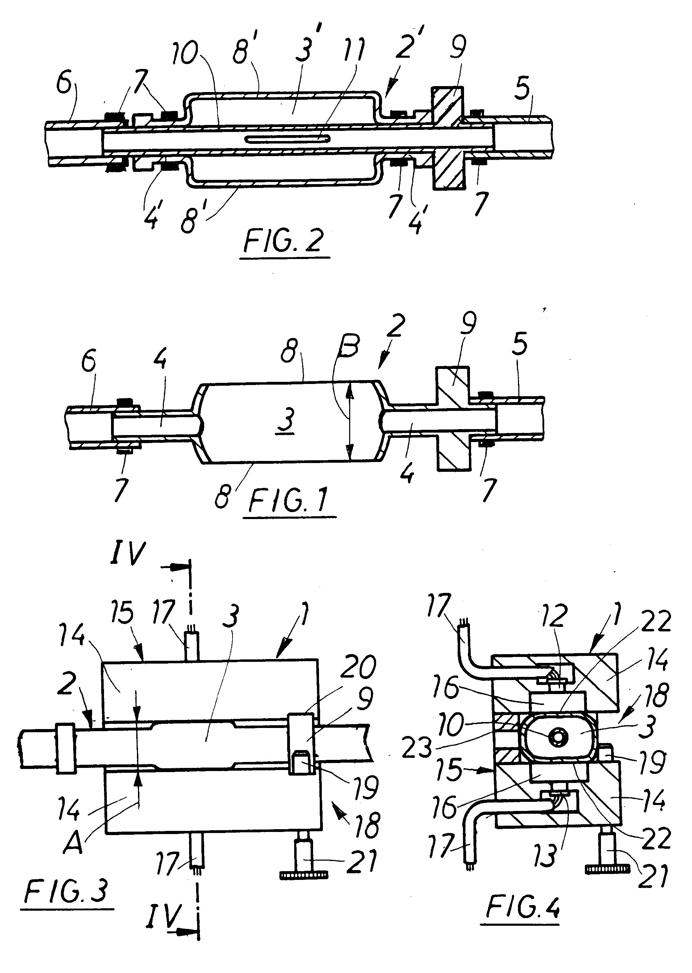

Die Druckmeßvorrichtung 1 umfaßt als wesentliches Bauteil ein Schlauchstück 2 mit einem Schlauchkissen 3 und zwei Schlauchenden 4. Diese sind in weitere Schlauchleitungen 5,6 festeingesetzt und durch Schlauchklemmen 7 fixiert. Das Schlauchkissen 3 besteht aus zwei parallel gegenüberliegenden Membranen 8 aus dünnem, gummielastischem Material von etwa 0,5 mm Dicke, wohingegen die Schlauchenden 4 eine Wandstärke von mindestens 1,5 mm aus Stabilitätsgründen haben. Die Membranen 8 sind somit hochelastisch. An einem Schlauchende 4 ist ein im wesentlichen rechteckiges Rastelement 9 angeformt, das aufgrund seiner Größe relativ starr ist,wie es in Figur 1 dargestellt ist .

In der zweiten, in der Fig. 2 dargestellten Ausführungsform ist das Schlauchstück 2′ mit seinen gegenüberliegenden und parallel zueinander angeordneten Membranen 8′ und seinen Schlauchenden 4′ im wesentlichen gleich ausgebildet wie das in Fig. 1 dargestellte Schlauchstück 2 der ersten Ausführungform. In der zweiten Ausführungform erstreckt sich durch beide Schlauchenden 4′ hindurch ein starres Innenrohr 10, das im Bereich der Membranen 8′ mit Längsschlitzen 11 zum Durchtritt des Fluides versehen ist. Die Schlauchenden 4′ sind mit Schlauchklemmen 7 auf das Innenrohr 10 aufgeklemmt, welches wiederum mit seinen Enden mit den Schlauchleitungen 5,6 über weitere Schlauchklemmen 7 verbunden ist. Das Rastelement 9′ ist bei der zweiten Ausführungsform einstückig aus dem Innenrohr 10 ausgeformt.

Ein durch das Schlauchstück 2,2′ strömendes Fluid beaufschlagt die Innenseiten der Membranen 8,8′ im Bereich des Schlauchkissens 3,3′ derart, daß ein Druck nach außen ausgeübt wird, der sich auf beide Membranen 8,8′ gleichmäßig auswirkt. Infolge der relativ dünnen und somit hochelastischen Membranen 8,8′ kann die durch unterschiedliche Fluiddrücke bewirkte Auswölbung der Membranen 8,8′ dazu verwendet werden, um mittels den Membranen 8,8′ zugeordneter Drucksensoren 12, 13 den Druck innerhalb des Schlauchstückes 2,2′ unabhängig voneinander an zwei Stellen zeit- und phasengleich zu messen. Dabei kann eine Druckmessung zur Ermittlung des tatsächlichen Druckes verwendet werden, wohingegen die zweite Druckmessung zur Drucküberwachung dient.

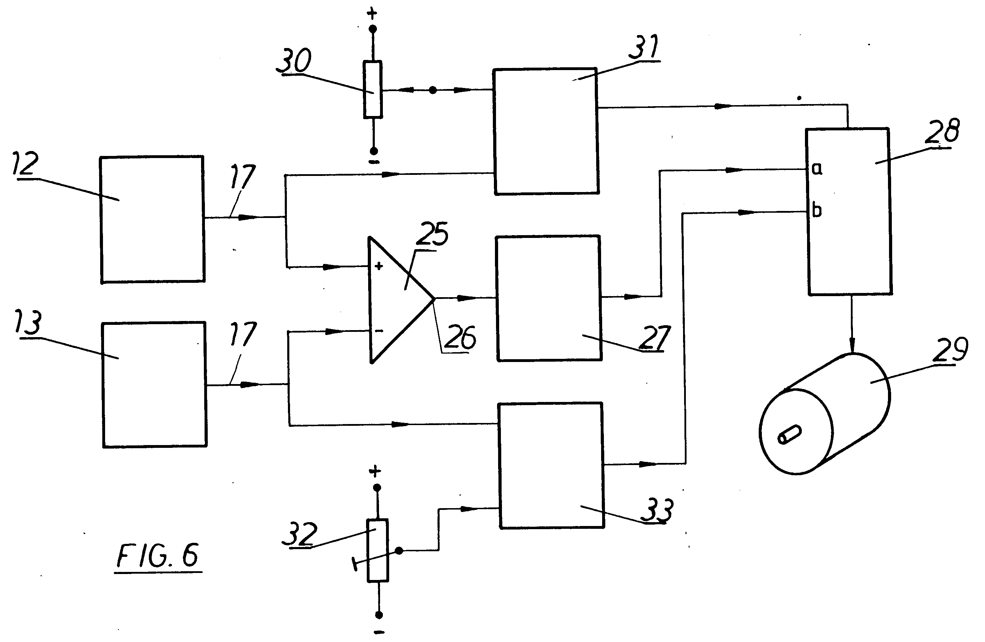

Die in den Figuren 3 und 4 näher dargestellte Druckmeßvorrichtung 1 umfaßt zur Aufnahme des Schlauchstückes 2, 2′ mit seinem Schlauchkissen 3,3′ zwei durch ein bodenseitiges Distanzstück 23 beabstandete, parallel angeordnete Stützwände 14, deren Abstand A voneinander genau der Breite B des Schlauchkissens 3,3′ im unbelasteten Zustand entspricht. Auf den Innenflächen der beiden eine Haltevorrichtung 15 bildenden Stützwände 14 sind gegenüberliegende Ausnehmungen 16 vorgesehen, deren Flächenabmessungen kleiner sind als die Flächenabmessungen der Membranen 8,8′. In die Außenwandflächen der Ausnehmungen 16 sind die Drucksensoren 12,13 eingesetzt, welche mit Zuleitungen 17 verbunden sind, die zu einer in Fig. 6 dargestellten Auswerteelektronik geführt sind. Die Ausnehmungen 16 sind durch dünne Metallmembranen 22 geschlossen und mit Drucköl vollständig ausgefüllt . Wie es die Figuren 3 und 4 zeigen, befindet sich das Schlauchstück 2,2′ mit seinem Schlauchkissen 3,3′ exakt zwischen den beiden Stützwänden 14 der Haltevorrichtung 15, wobei die Schlauchkissen 3,3′ zur Druckübertragung genau an den dünnen Metallmembranen 22 anliegen . In den Ausnehmungen 16 bilden sich Druckölsäulen aus, die zur Übertragung von Druckschwankungen von den Membranen 8,8′, welche die Ausnehmungen 16 abdecken,auf die Drucksensoren 12,13 dienen .

Zur Lagesicherung des Schlauchkissens 3 innerhalb der Meßvorrichtung 15 derart, daß die Gummi-Membranen 8,8′ genau mit den Metallmembranen 22 fluchten und diese abdecken , ist eine Rastvorrichtung 18 vorgesehen, die aus dem Rastelement 9 des Schlauchstückes 2 und einer Rastnase 19 gebildet ist, die das in eine Führung 20 innerhalb der Stützwände 14 eingeführte Rastelement 9 sichtbar gegen eine Verschiebung gesichert. Der Abstand zwischen dem Rastelement 9 und der Mittelachse des Schlauchkissens 3 ist dabei so gewählt, daß dieses mit seiner Membran 8 bei eingerastetem Rastelement 9 genau die Metallmembranen 22 abdeckt. Die Rastnase 19 wird durch einen federbelasteten Handgriff 21 betätigt.

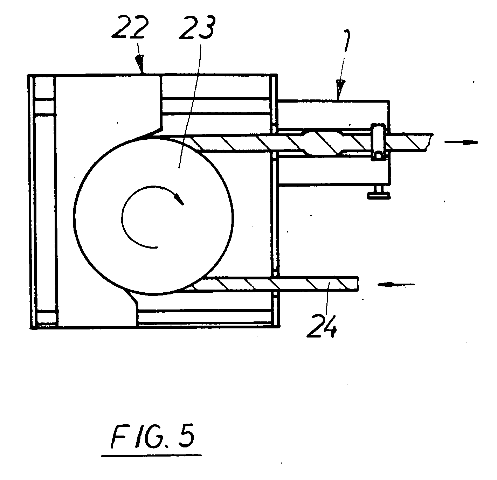

Wie es die Fig. 5 zeigt, wird die Druckmeßvorrichtung 1 mit einer Rollenpumpe 22 derart verbunden, daß die Druckmeßvorrichtung 1 auf der Ausgangsseite eines um die Rolle 23 herumgelegten Schlauches 24 angeordnet ist, wobei das Schlauchstück 2,2′ ein integrierendes Bestandteil des Schlauches 24 oder ein selbständiges Bestandteil sein kann . Die Figur 5 zeigt ferner , daß der Benutzer zugleich mit der Festlegung des Pumpenschlauches 24 gezwungen ist , das Schlauchkissen 3,3′ innerhalb der beiden Stützwände 14 mittels der Rastvorrichtung 18 genau einzuspannen.

Die Figur 6 zeigt schematisch das Schaltbild der Auswerteelektronik für die Druckmeßvorrichtung 1. Hierbei sind die beiden Drucksensoren 12,13, die mit eigenen Verstärkern versehen sind, mit einem Differenzverstärker 25 verbunden, an dessen Ausgang 26 der Differenzdruck erscheint, der gegebenenfalls zwischen den mittels der Drucksensoren 12, 13 gemessenen Druckwerten besteht. Dieser Differenzdruck wird einem Spannungsbereichdetektor 27 eingegeben, der feststellt, ob der Differenzdruck in einem erlaubten Bereich ist oder nicht. Der Ausgangswert des Spannungsbereichsdetektors 27 wird an einen Not-Stop-Eingang a der Steuerelektronik 28 des Pumpenmotors 29 gegeben. In der Auswerteelektronik ist ferner ein Sollwert-Istwertvergleicher(Komparator) 31 für den vorgewählten Systemdruck vorgesehen , der mit einem Sollwertgeber 30 und mit dem einen Drucksensor 12 verbunden ist. Der Ausgangswert wird ebenfalls auf die Steuerelektronik 28 für den Pumpenmotor 29 gegeben, um die Drucksteuerung der Rollenpumpe 22 zu bewirken. Schließlich ist in der Auswerteelektronik ein Einstellpotentiometer 32 für die Überdruckabschaltung vorgesehen, das mit einem Istwert-Maximalwertvergleicher 33 verbunden ist, dessen weiterer Eingang mit dem zweiten Drucksensor 13 verbunden ist. Der Ausgang des Istwert-Maximalwertvergleichers 33 ist mit dem zweiten Not-Stop-Eingang b der Steuerelektronik 28 des Motors 29 verbunden.

Die Druckmeßvorrichtung 1 arbeitet in Verbindung mit der Steuerelektronik gemäß Fig. 6 derart, daß der eine Drucksensor 12 seinen Druckwert einerseits unmittelbar über den Sollwert-Istwertvergleicher 31 zur Steuerelektronik 28 des Motors 29 der Rollenpumpe 22 liefert, um diese in Abhängigkeit vom mittels des Drucksensors 12 gemessenen und vom Sollwertgeber 30 vorgewählten Systemdruck zu steuern. Der zweite Drucksensor 13 gibt seinen Druckwert direkt an den Istwert-Maximalwertvergleicher 33, der mit dem Einstellpotentiometer 32 für die Überdruckabschaltung verbunden ist. Der zweite Drucksensor 13 dient somit zur Drucküberwachung und bewirkt bei einem den Maximaldruck übersteigenden Druck eine Abschaltung des Motors 29 der Rollenpumpe 22.

Zusätzlich werden die Druckwerte beider Drucksensoren 12, 13 zur Durchführung einer Differenzdruckmessung über den Differenzverstärker 25 herangezogen. Sofern der ermittelte Differenzdruck zwischen den beiden Drucksensoren 12,13 den erlaubten Bereich übersteigt, was vom Spannungsbereichdetektor 27 überwacht wird, wird der Motor 29 der Rollenpumpe 22 über die angeschlossene Steuerelektronik 28 abgeschaltet.

Die Verwendung zweier, unabhängig voneinander messender Drucksensoren 12,13, die zur Auswertung in drei verschiedenen Druckbereichen herangezogen werden, ist ein wesentliches Element der beschriebenen Druckmeßvorrichtung.

Das weitere wesentliche Element und das Herzstück der Druckmeßvorrichtung ist jedoch das Schlauchkissen 3,3′ mit seinen beiden Membranen 8,8′ , die als hochelastische , dünne Gummi-Membranflächen ausgebildet sind und die an den Metallmembranen 22 der Ausnehmungen 16 anliegen , um die Druckänderungen auf die Drucksensoren 12,13 über die Druckölsäulen zu geben , die eine berührungslose Übertragung der Druckschwankungen von den Membranen 8,8′ auf die Drucksensoren 12,13 ermöglichen. Da die Schlauchstücke 2,2′ als Einwegprodukte hergestellt werden können und die übrigen Bauelemente der Druckmeßvorrichtung 1 nicht sterilisiert werden müssen, ist die Druckmeßvorrichtung 1 äußerst einfach in der Handhabung und preiswert in der Herstellung.

## Patentansprüche

1. Druckmeß- und Überwachungsvorrichtung für in Leitungen strömende Fluide, insbesondere für die Schlauchleitung (2,21) druckgesteuerter Rollenpumpen zur Verwendung in der Hysteroskopie, Arthroskopie und in der Urethroskopie, mit einem in die Leitung (2,21) eingesetzten Schlauchkissen und mit zwei Druckmeßelementen (12,13),
**dadurch gekennzeichnet**, daß
das Schlauchkissen (3,3′) aus zwei dünnen, im wesentlichen parallel zueinander und im Abstand voneinander angeordneten Membranen (8,8′) gebildet ist, wobei das Schlauchkissen (3,3′) ein Innenrohr (10) aufweist, das mit Austrittsöffnungen (11) zu den Membranen (8,8′) versehen und an beiden Enden mit den Schlauchenden (4) eines Schlauchstückes (2) fest verbunden ist, und daß jeder Membrane (8,8′) ein Druckmeßelement in Form eines Drucksensors (12,13) zugeordnet ist und daß der eine Drucksensor (12) zur Messung des Fluiddruckes und der andere Drucksensor (13) zur Meßwertüberwachung dient und daß beide Drucksensoren (12,13) an eine Auswerteelektronik angeschlossen sind.

2. Druckmeßvorrichtung nach Anspruch 1, dadurch gekennzeichnet, daß die beiden Drucksensoren (12,13) symmetrisch zur Fläche der Membrane (8,8′) angeordnet sind.

3. Druckmeßvorrichtung nach Anspruch 1, dadurch gekennzeichnet, daß für das die beiden dünnen Gummi-Membranen (8,8′) aufweisende Schlauchkissen (3,3′) eine Haltevorrichtung (15) vorgesehen ist, die zwei im Abstand der Membranen (8,8′) voneinander befindliche Stützwände (14) mit durch Metallmembranen (22) geschlossenen, mit Drucköl angefüllten Ausnehmungen (16) aufweist, und daß die Metallmembranen (22) in den Ausnehmungen (16) zentrisch zu den Gummi-Membranen (8,8′) angeordnet sind.

4. Druckmeßvorrichtung nach Anspruch 3, dadurch gekennzeichnet, daß in der Haltevorrichtung (15) eine Rastvorrichtung (18) für das Schlauchstück (2) vorgesehen ist.

5. Druckmeßvorrichtung nach Anspruch 4, dadurch gekennzeichnet, daß die Rastvorrichtung (18) aus einem dem mit dem Schlauchkissen (3,3′) versehenen Schlauchstück (2,2′) zugeordneten Rastelement (9,9′) und zwei Führungen (20) für das Rastelement (9,9′) in den Stützwänden (14) sowie mit einer Rastnase (19) versehen ist, welche das Rastelement (9,9′) in der Haltevorrichtung (15) sichert.

## Claims

1. A pressure measuring device for fluids flowing in pipes, in particular for the hose pipe (2, 21) of pressure-controlled roller pumps for application in the hysteroscopy, arthroscopy and urethroscopy, comprising a hose cushion inserted into the pipe (2, 21) and two pressure measuring elements (12, 13), characterized by that the hose cushion (3, 3′) is formed of two thin substantially parallel and spaced diaphragms (8, 8′), the hose cushion (3, 3′) comprising an internal tube (10) provided with outlet openings (11) to the diaphragms (8, 8′) and being firmly connected at both ends with the hose ends (4) of a hose portion (2), and that a pressure measuring element in the form of a pressure sensor (12, 13) is assigned to each diaphragm (8, 8′), one (12) of said pressure sensors serving for the measurement of the fluid pressure and the other (13) for the supervision of the measured value, and that both pressure sensors (12, 13) are connected to an evaluation circuit.

2. A pressure measuring device according to claim 1, characterized by that the two pressure sensors (12, 13) are disposed symmetrically to the surface of the diaphragm (8, 8′).

3. A pressure measuring device according to claim 1, characterized by that for the hose cushion (3, 3′) comprising the two thin rubber diaphragms (8, 8′) is provided a holding device (15) including two support walls (14) spaced to the diaphragms (8, 8′) and recesses (16) covered by the metal diaphragms (22) and filled-up with pressure oil, and that the metal diaphragms (22) are disposed in the recesses (16) centrally to the rubber diaphragms (8, 8′).

4. A pressure measuring device according to claim 3, characterized by that in the holding device (15) is provided a latch device (18) for the hose portion (2).

5. A pressure measuring device according to claim 4, characterized by that the latch device (18) is provided with a latch element (9, 9′) assigned to the hose portion (2, 2′) provided with the hose cushion (3, 3′) and with a latch lug (19) securing the latch element (9, 9′) in the holding device (15).

## Revendications

1. Dispositif de mesure et de contrôle de pression de fluides coulants dans des conduites, essentiellement pour la conduite flexible (2,21) de pompes à rouleaux commandées par pression utilisée dans la hystéroscopie, l'arthroscopie et l'uréthroscopie, avec un coussin tubulaire inséré dans la conduite (2,21) et avec deux éléments de mesure de pression (12,13),
**caractérisé en ce que**
le coussin tubulaire (3,3′) est formé de deux membranes (8,8′) minces, essentiellement parallèles l'une par rapport à l'autre et disposées à un certain écart l'une de l'autre, le coussin tubulaire (3,3′) comportant un tube intérieur (10) muni d'ouvertures de sortie (11) vers les membranes (8,8′) et relié avec les deux extrémités à demeure avec les extrémités (4) d'une pièce de tuyau (2), et qu'à chaque membrane (8,8′) est conjugué un élément de mesure de pression sous forme d'un palpeur de pression (12,13), et que l'un des palpeurs de pression (12) sert à la mesure de la pression du fluide et l'autre palpeur de pression (13) au contrôle de la valeur de mesure et que les deux palpeurs de pression (12,13) sont raccordés à une électronique d'évaluation.

2. Dispositif de mesure de pression selon la revendication 1, caractérisé en ce que les deux palpeurs de pression (12,13) sont disposés symétriquement à la surface de la membrane (8,8′).

3. Dispositif de mesure de pression selon la revendication 1, caractérisé en ce que, pour le coussin tubulaire (3,3′) comprenant les deux minces membranes en caoutchouc (8,8′), est prévu un dispositif d'arrêt (15) présentant deux parois d'appui (14) situées à un certain écart des membranes (8,8′) avec des évidements (16) fermés par des membranes métalliques (22) et remplis d'huile de pression, et que les membranes métalliques (22) dans les évidements (16) sont disposées de façon centrée par rapport aux membranes en caoutchouc (8,8′).

4. Dispositif de mesure de pression selon la revendication 3, caractérisé en ce que un dispositif de retenue (18) pour la partie de tuyau (2) est prévu dans le dispositif d'arrêt (15).

5. Dispositif de mesure de pression selon la revendication 4, caractérisé en ce que le dispositif de retenue (18) comporte un élément de retenue (9,9′) conjugué à la pièce de tuyau (2,2′) muni du coussin tubulaire (3,3′) et deux guidages (20) pour l'élément de retenue (9,9′) dans les parois d'appui (14), ainsi qu'un talon d'arrêt (19) bloque l'élément de retenue (9,9′) dans le dispositif d'arrêt (15).
